# EUROPEAN PATENT APPLICATION

(11) **EP 1 262 197 A2**
(43) Date of publication of application: **04.12.2002**
(21) Application number: 02253589.2
(22) Date of filing: 22.05.2002
(51) Int. Cl.: A61K 45/06, A61P 25/20

(54) **Combination treatment for sleep disorders including sleep apnea**

(30) Priority: 30.05.2001 US 294322 P
(71) Applicant: Pfizer Products Inc., Groton, Connecticut 06340 (US)
(72) Inventor: Howard, Harry Ralph, Jr., Pfizer Global Research, Groton, Connecticut 06340 (US)
(74) Representative: Sewell, Richard Charles

(57) **Abstract**

The present invention relates to a method of treating sleep disorders including sleep apnea in a mammal, including a human, by administering to the mammal a 5HT1a antagonist or an alpha-2-adrenergic antagonist in combination with an SRI antidepressant agent with improvement in efficacy. It also relates to pharmaceutical compositions containing a pharmaceutically acceptable carrier, a 5HT1a antagonist or an alpha-2-adrenergic antagonist, and an SRI antidepressant agent.

## Description

### Background Of The Invention

The present invention relates to a method of treating sleep disorders including sleep apnea with improved efficacy in a mammal, including a human, by administering to the mammal a 5HT1a antagonist or an alpha-2-adrenergic antagonist in combination with a serotonin reuptake inhibitor (SRI). It also relates to pharmaceutical compositions containing a pharmaceutically acceptable carrier, a serotonin 5HT1a antagonist or an alpha-2-adrenergic antagonist and a serotonin reuptake inhibitor (SRI).

Sleep disorders including sleep apnea which are to be treated according to the present invention are of a psychiatric nature, and are to be diagnosed, and the treatment prescribed, by psychiatrists and other physicians. It will be understood that the patient and doctor cannot expect that such treatment will effect a cure in all cases. However, treatment according to the present invention, perhaps combined with other treatments such as psychiatric consultation and analysis, lifestyle modification, and perhaps other treatments for concomitant disorders, will be found to alleviate the disorder of sleep, producing a substantial benefit to the patient. In some cases, the benefit will be in the form of an alleviation of the unpleasant symptoms of the disorders, and in other cases substantial or even complete diminution of the symptoms will be obtained, amounting to complete cure of the disorder.

Serotonin Selective Reuptake Inhibitors (SSRIs) currently provide efficacy in the treatment of major depressive disorder (MDD) and are generally perceived by psychiatrists and primary care physicians as effective, well-tolerated and easily administered. However, they are associated with undesirable features, such as high incidence of sexual dysfunction, delayed onset of action and a level of non-responsiveness estimated to be as high as 30% (see M. J. Gitlin, Journal of Clinical Psychiatry, **1994,** 55, 406-413 and R. T. Segraves, Journal of Clinical Psychiatry, **1992,** 10(2), 4-10). Preclinical and clinical evidence has indicated that the sexual dysfunction associated with SSRI therapy can be reduced through the use of serotonin reuptake inhibitors (SRI) and dopamine reuptake inhibitors (DRIs), such as bupropion (see A. K. Ashton, Journal of Clinical Psychiatry , **1998,** 59(3), 112-115). Furthermore, the combination of SRI and DRI may hasten the onset of action as well as offering relief to refractory patients, possibly through a synergistic mechanism (see R. D. Marshall et al, Journal of Psychopharmacology, **1995,** 9(3), 284-286) and prove beneficial in the treatment of substance abuse and attention deficit hyperactivity disorder (ADHD) according to Barrickman et al, Journal of the American Academy of Child and Adolescent Psychology, **1995,** 34(5), 649 and Shekim et al, Journal of Nervous and Mental Disease, **1989,** 177(5), 296.

### Summary Of The Invention

The present invention relates to a pharmaceutical composition for the treatment of sleep disturbances, including apnea comprising: (a) a compound that exhibits activity as a Serotonin Reuptake Inhibitor, or a pharmaceutically acceptable salt thereof; (b) a 5HT1a antagonist or an alpha-2-adrenergic antagonist or pharmaceutically acceptable salt thereof; and (c) a pharmaceutically acceptable carrier; wherein the active agents "a" and "b" above are present in amounts that render the composition effective in treating, respectively, sleep disturbances including sleep apnea refractory to treatment with traditional sleep medication alone.

This invention also relates to a method of treating sleep disturbances including sleep apnea in a mammal, comprising administering to said mammal, respectively, an anti-sleep disturbance effective amount of a pharmaceutical composition comprising: (a) a Serotonin Reuptake Inhibitor (SRI) compound that exhibits activity as an antidepressant, or a pharmaceutically acceptable salt thereof; (b) a 5HT1a antagonist or an alpha-2-adrenergic antagonist or pharmaceutically acceptable salt thereof; and (c) a pharmaceutically acceptable carrier; wherein the active agents "a" and "b" above are present in amounts that render the composition effective in treating, respectively, sleep disturbances including sleep apnea with improvement in the efficacy achieved by either component individually.

This invention also relates to a method of treating sleep disturbances including sleep apnea in a mammal, comprising administering to said mammal: (a) a Serotonin Reuptake Inhibitor (SRI) compound that exhibits activity as, respectively an antidepressant, or a pharmaceutically acceptable salt thereof; and (b) a 5HT1a antagonist or an alpha-2-adrenergic antagonist or pharmaceutically acceptable salt thereof; wherein the active agents "a" and "b" above are present in amounts that render the combination of the two agents effective in treating, respectively, sleep disturbances including sleep apnea with improvement in the efficacy achieved by either component individually in the treatment of sleep disturbances, especially sleep apnea.

It will be appreciated that when using a combination method of the present invention, referred to immediately above, both the 5HT1a antagonist or the alpha-2-adrenergic antagonist and the SRI antidepressant will be administered to a patient within a reasonable period of time. The compounds may be in the same pharmaceutically acceptable carrier and therefore administered simultaneously. They may be in separate pharmaceutical carriers such as conventional oral dosage forms that are taken simultaneously. The term combination, as used above, also refers to the case where the compounds are provided in separate dosage forms and are administered sequentially. Therefore, by way of example, the SRI antidepressant agent may be administered as a tablet and then, within a reasonable period of time, the 5HT1a antagonist or an alpha-2-adrenergic antagonist may be administered either as an oral dosage form such as a tablet or a fast-dissolving oral dosage form. By a "fast dissolving oral formulation" is meant, an oral delivery form which when placed on the tongue of a patient, dissolves within about seconds

The compositions of the present invention that contain a 5HT1a antagonist or an alpha-2-adrenergic antagonist and an SRI antidepressant are useful for the treatment of sleep disturbances including apnea.

Specific disorders of sleep to be treated according to the present invention will be described according to the nomenclature in the Diagnostic and Statistical Manual of Mental Disorders. 4th Edition (1994), published by the American Psychiatric Association. Sleep disorders which are of particular interest with relation to the present invention are primary insomnia (DSM-IV Code 307.42), primary hypersomnia (307.44), narcolepsy (347), circadian rhythm sleep disorder (307.45). Parasomnias including nightmare disorder (307.47), sleep, terror disorder (307.46), and sleepwalking disorder (307.46), sleep disorders related to another mental disorder (307.42 and 307.44), sleep disorders due to a general medical condition (780.xx) and substance-induced sleep disorders.

Further description and discussion of sleep disorders are found in the International Classification of Sleep Disorders: Diagnostic and Coding Manual (1990), published by the American Sleep Disorders Association.

No doubt the best known disorder of sleep is primary insomnia, the difficulty in initiating or maintaining sleep, sometimes also manifested by the patient's being asleep but not being rested or restored. Most often patients report a combination of difficulty falling asleep and intermittent wakefulness, during sleep. A preoccupation with and distress due to the inability to sleep may create a cycle; the more the patient strives to sleep, the more frustrated the individual becomes and the less he or she is able to sleep. Chronic insomnia may lead to decreased feelings of well-being during the day, with decreased attention, energy and concentration and an increase in fatigue. Personal, social and occupational problems may develop and patients may have accidents. The sleep disturbance constitutes a risk factor for subsequent mood disorders and anxiety disorders, as well as a risk factor for inappropriate use of hypnotics, alcohol, anxiolytics, caffeine and other stimulants. The true prevalence of primary insomnia among the general population is unknown, but may be quite high. About 15-25% of patients presenting to sleep clinics complaining of insomnia are found to have primary insomnia.

The DSM-IV lists the diagnostic criteria for primary insomnia as follows:
A. The predominant complaint is difficulty initiating or maintaining sleep, or nonrestorative sleep, for at least 1 month.
B. The sleep disturbance (or associated daytime fatigue) causes clinically significant distress or impairment in social, occupational: or other important areas of functioning.
C. The sleep disturbance does not occur exclusively during the course of Narcolepsy, Breathing-Related Sleep Disorder, Circadian Rhythm Sleep Disorder, or a Parasomnia.
D. The disturbance does not occur exclusively during the course of another mental disorder (e.g., Major Depressive Disorder. Generalized Anxiety Disorder, a delirium).
E. The disturbance is not due to the direct physiological effects of a substance (e.g., a drug of abuse; a medication) or a general medical condition.

Primary hypersomnia is evidenced by excessive sleepiness in the form of either prolonged sleep episodes or by frequent daytime sleep episodes. The excessive sleepiness is sufficiently severe to cause distress or impairment in social, occupational and other important aspects of the patient's life. Such patients sleep from 8-12 hours every night, and often have difficulty awakening. Daytime naps tend to be relatively long as well, and are not refreshing. Hypersomnia patients' daytime sleep episodes can be embarrassing and even dangerous, if the individual is operating a machine for example, and the patient's low alertness leads to poor efficiency and other difficulties during daytime activities

Of course, the normal range of sleep duration varies considerably. Individuals who naturally require a relatively large amount of sleep, but do not have excessive daytime sleepiness, are not suffering from hypersomnia, and the diagnosis is readily made, the diagnostic criteria for hypersomnia are as follows:
A. The predominant complaint is excessive sleepiness for at least 1 month (or less if recurrent) as evidenced by either prolonged sleep episodes or daytime sleep episodes that occur almost daily
B. The excessive sleepiness causes clinically significant distress or impairment in social, occupational, or other important areas of functioning.
C. The excessive sleepiness is not better accounted for by insomnia and does not occur exclusively during the course of another Sleep Disorder (e.g. Narcolepsy,: Breathing-Related Sleep Disorder, Circadian Rhythm Sleep Disorder or a Parasomnia) and cannot be accounted for by an inadequate amount of sleep.
D. The disturbance does not occur exclusively during the course of another mental disorder.
E. The disturbances are not due to the direct physiological effects of a substance (e.g., a drug of abuse, a medication) or a general medical condition.

Narcolepsy is characterized by repeated attacks of refreshing sleep, usually accompanied with cataplexy. Episodes of sleepiness are often irresistible, resulting in falling asleep while driving or carrying on a conversation. Sleep episodes are- usually brief but can last up to an hour, and frequently recur 2-6 times per day.

Patients with narcolepsy may avoid social activities and their functioning of all kinds can be severely limited and impaired. Patients are at considerable risk of injury because of falling asleep in dangerous situations.

The degree of daytime sleepiness may be similar in patients with narcolepsy and primary hypersomnia, but narcolepsy patients have more urgent sleep attacks. Cataplexy, sleep-related hallucinations and sleep paralysis are confined to narcolepsy patients. The diagnostic criteria for narcolepsy are as follows:
A. Irresistible attacks of refreshing sleep that occur daily over at least 3 months.
B. The presence of one or both of the following:
   (1) cataplexy (i.e., brief episodes of sudden bilateral loss of muscle tone. most often in association with intense emotion).
   (2) recurrent intrusions of elements of rapid eye movement (REM) sleep into the transition between sleep and wakefulness, as manifested by either hypnopompic or hypnagogic hallucinations or sleep paralysis at the beginning or end of sleep episodes.
C. The disturbance is not due to the direct physiological effects of a substance (e.g., a drug of abuse, a medication) or another general medical condition.

Circadian rhythm sleep disorder does not result directly from the mechanisms generating sleep and wakefulness, but is a pattern of sleep disruption resulting from incongruity between the patient's needs to maintain a schedule, and his or her internal sleep-waking system. Individuals with the disorder may be excessively sleepy at some times of the day and complain of insomnia at other times.

Circadian rhythm sleep disorder may be of the jet lag type, which is self-explanatory, the shift work type, or the delayed sleep phase type wherein the patient's sleep-wake cycle is delayed relative to the needed schedule. Such individuals are chronically sleep-deprived but their sleep is normal once it is initiated. The familiar people who are, morning type" and "night owls" have a circadian rhythm disorder which in effect deprives them of part of a normal waking day. The diagnostic criteria for circadian rhythm sleep disorder are as follows:
A. A persistent or recurrent pattern of sleep disruption leading to excessive sleepiness or insomnia that is due to a mismatch between the sleep-wake schedule required by a person's environment and his or her circadian sleepwake pattern.
B. The sleep disturbance causes clinically significant distress or impairment in social, occupational, or other important areas of functioning.
C. The disturbance does not occur exclusively during the course of another Sleep Disorder or other mental disorder
D. The disturbance is not due to the direct physiological effects of a substance (e.g., a drug of abuse, a medication) or a general medical condition.

Parasomnias are disorders which are brought about by activation of inappropriate sections of the nervous system during sleep or sleep-waking transitions. Parasomnias include nightmare disorder, sleep terror disorder and sleepwalking disorder, the unpleasant nature of which are described by their mere names. The diagnostic criteria for these disorders further describe the disruption which they cause:

### Nightmare Disorder

A. Repeated awakenings from the major sleep period or naps with detailed recall of extended and extremely frightening dreams, usually involving threats to survival, security, or self-esteem. The awakenings generally occur during the second half of the sleep period.
B. On awakening from the frightening dreams, the person rapidly becomes oriented and alert (in contrast to the confusion and disorientation seen in Sleep Terror Disorder and some forms of epilepsy.).
C. The dream experience, or the sleep disturbance resulting from the awakening, causes clinically significant distress or impairment in social, occupational, or other important areas of functioning.
D. The nightmares do not occur exclusively during the course of another mental disorder (e.g., a delirium, Posttraumatic Stress Disorder) and are not due to the direct physiological effects of a substance (e.g., a drug of abuse, a medication) or a general medical condition.

### Sleep Terror Disorder

A. Recurrent episodes of abrupt awakening from sleep, usually occurring during the first third of the major sleep episode and beginning with a panicky scream.
B. Intense fear and signs of autonomic arousal, such as tachycardia, rapid breathing and sweating during each episode.
C. Relative unresponsiveness to the efforts of others to comfort the person during the episode.
D. No detailed dream is recalled and there is amnesia for the episode.
E. The episodes cause clinically significant distress or impairment in social, occupational, or other important areas of functioning.
F. The disturbance is not due to the direct physiological effects of a substance (e.g., a drug of abuse, a medication) or a general medical condition.

### Sleepwalking Disorder

A. Repeated episodes of rising from bed during sleep and walking about, usually occurring during the first third of the major sleep episode.
B. While sleepwalking, the person has a blank, staring face, is relatively unresponsive to the efforts of others to communicate with him or her andcan be awakened only with great difficulty.
C. On awakening (either from the sleepwalking episode or the next morning), the person has amnesia for the episode.
D. Within several minutes after awakening from the sleepwalking episode, there is no impairment of mental activity or behavior (although there may initially be a short period of confusion or disorientation).
E. The sleepwalking causes clinically significant distress or impairment in social, occupational, or other important areas of functioning.
F. The disturbance is not due to the direct physiological effects of a substance (e.g., a drug of abuse, a medication) or a general medical condition.

Disorders of sleep frequently occur in relation to, or because of, another mental disorder or a general medical condition. Both insomnia and hypersomnia frequently are related to such other conditions. The symptoms with which the patient presents in such disorders are substantially the same as the symptoms of primary insomnia or primary hypersomnia, but the patient's history and other diagnoses bring out the relation to the other mental or general medical conditions,

The following diagnostic criteria illustrate the circumstances of patients with insomnia or hypersomnia related to another mental disorder:

### Insomnia Related to Axis I or Axis II disorder

A. The predominant complaint is difficulty initiating or maintaining sleep, or nonrestorative sleep, for at least 1 month that is associated with daytime fatigue or impaired daytime functioning.
B. The sleep disturbance (or daytime sequelae) causes clinically significant distress or impairment in social, occupational or other important area's of functioning.
C. The insomnia is judged to be related to another Axis-I or Axis II disorder (e.g., Major Depressive Disorder, Generalized Anxiety Disorder, Adjustment Disorder With Anxiety), but is sufficiently severe to warrant independent clinical attention.
D. The disturbance is not better accounted for by another Sleep Disorder (e.g., Narcolepsy, Breathing- Related Sleep Disorder, a Parasomnia).
E. The disturbance is not due to the direct physiological effects of a substance (e.g.: a drug of abuse, a medication) or a general medical condition.

### Hypersomnia Related toAxis I or Axis II disorder

A. The predominant complaint is excessive sleepiness for at least 1 month as evidenced by either prolonged sleep episodes or daytime sleep episodes that occur almost daily.
B. The excessive sleepiness causes clinically significant distress or impairment in social, occupational, or other important areas of functioning.
C. The hypersomnia is judged to be related to another Axis I or Axis II disorder (e.g., Major Depressive Disorder, Dysthymic Disorder), but is sufficiently severe to warrant independent clinical attention.
D. The disturbance is not better accounted for by another Sleep Disorder (e.g. Narcolepsy, Breathing-Related Sleep Disorder, a Parasomnia) or by an inadequate amount of sleep.
E. The disturbance is not due to the direct physiological effects of a substance (e.g., a drug of abuse, a medication) or a general medical condition.

The following diagnostic criteria illustrate insomnia or hypersomnia related to, or due to, a general medical condition.
A. A prominent disturbance in sleep that is sufficiently severe to warrant independent clinical attention.
B. There is evidence from the history, physical examination, or laboratory findings that the sleep disturbance is the direct physiological consequence of a general medical condition.
C. The disturbance is not better accounted for by another mental disorder (e.g., an Adjustment Disorder in which the stressor is a serious medical illness).
D. The disturbance does not occur exclusively during the course of a delirium.
E. The disturbance does not meet the criteria for Breathing-Related Sleep Disorder or Narcolepsy.
F The sleep disturbance causes clinically significant distress or impairment in, social, occupational, or other important areas of functioning.

Disorders of sleep, finally, may be induced by inappropriate use of substances, such as alcohol, drugs of abuse, or pharmaceuticals. Amphetamines, caffeine, cocaine, opioids, sedatives, hypnotics and anxiolytics may be associated with substance-induced sleep disorders. Such sleep disorders can occur during intoxication, during withdrawal from the substance, or both. Both insomnia and hypersomnia are found in patients with substance-induced sleep disorders. The treatment of substance-induced sleep disorders (as well as that of sleep disorders due to a general medical condition) may be complicated by treatment of the substance addiction or the medical condition with drugs which cause or exacerbate a sleep complaint in themselves. The following diagnostic criteria more precisely describes substance-induced sleep disorder.
A. A prominent disturbance in sleep that is sufficiently severe to warrant independent clinical attention.
B. There is evidence from the history, physical examination, or laboratory findings of either (1) or (2):
   (1) the symptoms in Criterion A developed during, or within a month of substance intoxication or withdrawal
   (2) medication use is etiologically related to the sleep disturbance
C. The disturbance is not better accounted for by a Sleep Disorder that is not substance induced. Evidence that the symptoms are better accounted for by a Sleep Disorder that is not substance induced might include the following: the symptoms precede the onset of the substance use (or medication use); the symptoms persist for a substantial period of time (e.g., about a month) after the cessation of acute withdrawal or severe intoxication, or they are substantially in excess of what would be expected given the type or amount of the substance used or the duration of use, or there is other evidence that suggests the existence of an independent non-substance-induced Sleep Disorder (e.g., a history of recurrent non-substance-related episodes).
D. The disturbance does not occur exclusively during the course of a delirium.
E. The sleep disturbance causes clinically significant distress or impairment in social, occupational, or other important areas of functioning.

The present invention also pertains to the treatment of sleep apneas. Sleep apnea is defined as the cessation of breathing during sleep. It comprises a spectrum of respiration related disorders with varying severity and morbidity involving periods, during sleep, in which airflow is disturbed. The usual classification of sleep apneas distinguishes obstructive, central, and mixed apneas, depending on the presence or absence of respiratory efforts during the periods in which airflow has ceased. In the case of the obstructive sleep apnea syndrome, which is the most familiar apnea, sporadic recurring collapse of the patient's upper airway occurs during sleep. If the collapse is complete, there is no air exchange at the nose and the mouth, and breathing is interrupted. The usual result is a partial arousal from sleep and a return to normal breathing. The patient in most instances does not have any knowledge or memory of these apnea episodes, but finds himself constantly suffering from fatigue and daytime sleepiness for no apparent reason. These recurrent apnea episodes with resultant hypoxemia and fragmented sleep can have serious neurologic and cardiac consequences. While the obstructive sleep apnea is a physical blockade, central sleep apnea is defined as a neurological disorder, causing cessation of all respiratory effort during sleep, usually with decreases in blood oxygen saturation. The effects of both types of apneas are highly similar. Mixed apnea is a combination of the previous two. An episode of mixed sleep apnea usually starts with a central component and then becomes obstructive in nature.

The sleep apnea syndrome today is regarded as a serious problem, as it occurs widely and there is a true lack of an effective treatment. Surgical and mechanical interventions have been suggested and tried as treatments, as has oxygen administration during sleep, but none of these are recognized to be very suitable. Pharmacological intervention has also been tried, but with little success. In fact, several kinds of respiratory stimulants, theophylline, antidepressants, and progestogens have been used to treat sleep apneas, but none of these has been found to be very effective.

The compositions of the present invention are especially useful for the treatment of sleep disorders including sleep apnea where the use of an antidepressant is generally prescribed. By the use of a combination of a 5HT1a antagonist or an alpha-2-adrenergic antagonist and an SRI antidepressant agent in accordance with the present invention, it is possible to treat sleep disorders including sleep apnea in patients for whom conventional psychiatric therapy might not be wholly successful or where a faster onset of action is needed.

The term "treating", as used herein, refers to reversing, alleviating, inhibiting the progress of, or preventing the disorder or condition to which such term applies, or one or more symptoms of such condition or disorder. The term "treatment", as used herein, refers to the act of treating, as "treating" is defined immediately above.

Examples of Serotonin Reuptake Inhibitors (SRI) that may be used in the methods and pharmaceutical compositions of this invention are compounds of the formula 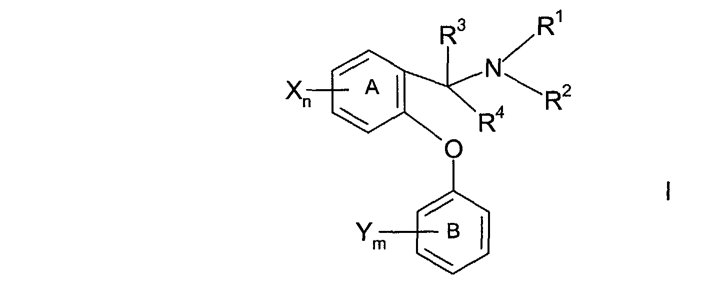 wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³ , together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X and each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl wherein R⁵ is hydrogen or (C₁-C₆)alkyl, and SOₚ(C₁-C₆)alkyl wherein p is zero, one or two; and
with the proviso that: (a) no more than one of NR¹R², CR³R⁴ and R²NCR³ can form a ring; and (b) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups; and the pharmaceutically acceptable salts thereof.

Pharmaceutically acceptable acid addition salts of the compounds of formula I can also be used in the methods and pharmaceutical composition of this invention. Examples of pharmaceutically acceptable acid addition salts of the compounds of formula I are the salts of hydrochloric acid, p-toluenesulfonic acid, fumaric acid, citric acid, succinic acid, salicylic acid, oxalic acid, hydrobromic acid, phosphoric acid, methanesulfonic acid, tartaric acid, maleic acid, di-p-toluoyl tartaric acid, acetic acid, sulfuric acid, hydroiodic acid and mandelic acid.

Unless otherwise indicated, the term "halo", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

The compounds of formula I may have optical centers and therefore may occur in different enantiomeric configurations. All enantiomers, diastereomers, and other stereoisomers of such compounds of formula I, as well as racemic and other mixtures thereof are included in the pharmaceutical compositions and methods of this invention.

The pharmaceutical compositions and methods of this invention also relates to all radiolabelled forms of the compounds of the formula I. Preferred radiolabelled compounds of formula I are those wherein the radiolabels are selected from ³H, ¹¹C, ¹⁴C, ¹⁸F, ¹²³I and ¹²⁵I. Such radiolabelled compounds are useful as research and diagnostic tools in metabolism pharmacokinetics studies and in binding assays in both animals and man.

"Chemical dependency," as used herein, means an abnormal craving or desire for, or an addiction to a drug. Such drugs are generally administered to the affected individual by any of a variety of means of administration, including oral, parenteral, nasal or by inhalation. Examples of chemical dependencies treatable by the methods of the present invention are dependencies on alcohol, nicotine, cocaine, heroin, phenobarbital, and benzodiazepines (e.g., Valium (trademark)). "Treating a chemical dependency," as used herein, means reducing or alleviating such dependency.

Preferred embodiments of formula I include the following compounds of the formula I and their pharmaceutically acceptable salts:
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine; and
{1-[2-(4-Chlorophenoxy)-5-fluorophenyl]-ethyl}-methylamine.
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichiorophenoxy)-5-fluorophenyi]-N-methyipyrrolidine.

Other embodiments of formula I include the following compounds and their pharmaceutically acceptable salts:
{1-[2-(3,4-Dichlorophenoxy)-5-fluorophenyl]-1-methylethyl}-methylamine;
{1-[2-(3,4-Dichlorophenoxy)-5-fluorophenyl]-1-methylethyl}-dimethylamine;
[4-Chloro-2-(4-chlorophenoxy)-5-fluorobenzyl]-methylamine;
[2-(3,4-Dichlorophenoxy)-5-fluoro-4-methoxybenzyl]-methylamine;
[4-(3,4-Dichlorophenoxy)-3-(dimethylaminomethyl)-phenyl]-dimethylamine
[5-Fluoro-2-(4-fluoro-3-methoxyphenoxy)-benzyl]-dimethylamine;
[2-(4-Chlorophenoxy)-5-isopropylbenzyl]-methylamine;
{1-[2-(4-Chlorophenoxy)-5-trifluoromethylphenyl]-ethyl}-methylamine;
[2-(4-Chlorophenoxy)-4,5-dimethylbenzyl]-methylamine;
{1-[5-Chloro-2(3,4-dichlorophenoxy)phenyl]-propyl}-methylamine;
[2-(3,4-Dichlorophenoxy)-5-methylsulfanyl-benzyl]-methylamine;
{1-[2-(3,4-Dichlorophenoxy)-5-methylsulfanyl-phenyl]-ethyl}-methylamine;
{1-[2-(3,4-Dichloro-phenoxy)-5-methylsulfanyl-phenyl]-1-methylethyl}-methylamine;
[2-(3,4-Dichlorophenoxy)-5-methylsulfanyl-benzyl]-dimethylamine;
[2-(3,4-Dichlorophenoxy)-5-methanesulfinyl-benzyl]-dimethylamine ;
[2-(3,4-Dichlorophenoxy)-5-methanesulfinyl-benzyl]-methylamine;
[2-(3,4-Dichlorophenoxy)-5-methanesulfonyl-benzyl]-methylamine;
[2-(3,4-Dichlorophenoxy)-5-methanesulfonyl-benzyl]-dimethylamine;
[2-(3,4-Dichlorophenoxy)-5-(propane-2-sulfonyl)-benzyl]-methylamine;
2-[2-(3,4-Dichlorophenoxy)-5-fluorophenyl]-piperidine;
2-[2-(3,4-Dichlorophenoxy)-5-fluorophenyl]-1-methyl-piperidine;
3-[2-(3,4-Dichlorphenoxy)-5-fluorophenyl]-4-methyl-morpholine;
2-[2-(3,4-Dichlorophenoxy)-5-fluorophenyl]-1,2-dimethyl-piperidine;
{1-[2-(3,4-Dichlorophenoxy)-5-fluorophenyl]-cyclopropyl}-dimethylamine;
2-[2-(3,4-Dichlorophenoxy)-5-fluorophenyl]-1,5-dimethyl-pyrrolidine;
3-[2-(3,4-Dichlorophenoxy)-5-fluorophenyl]-4-methyl-thiomorpholine;
{1-[2-(3,4-Dichlorophenoxy)-5-fluorophenyl]-cyclopentyl}-methylamine;
{1-[2-(3,4-Dichlorophenoxy)-5-(propane-2-sulfonyl)-phenyl]-ethyl}-methylamine; and
[4-Chloro-2-(3,4-dichlorophenoxy)-5-methanesulfonyl-benzyl]-methylamine.

One embodiment of this invention relates to compositions wherein the SRI antidepressant is a compound of formula I wherein n is one, X is fluoro, R³ and R⁴ are hydrogen, R¹ is hydrogen, R² is methyl, m is two and Yₘ is 3,4-dichloro.

Another embodiment of this invention relates to compositions wherein the SRI antidepressant is a compound of the formula I wherein m is zero, n is one, R³ and R⁴ are hydrogen, X is chloro, bromo, iodo or methyl, R¹ is hydrogen and R² is methyl.

Other examples of Serotonin Reuptake Inhibitors (SRI) that can be used in the method and pharmaceutical compositions of this invention are compounds of the formula 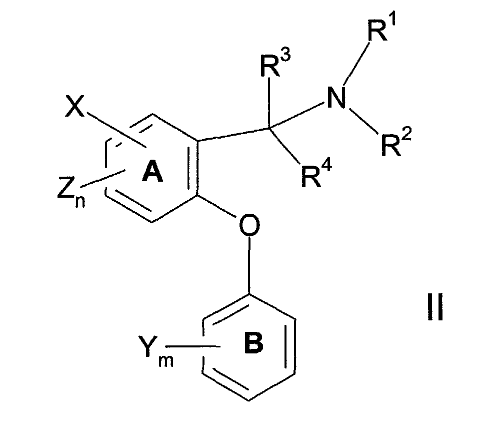 wherein phenyl ring A and phenyl ring B can each, independently, be replaced by a naphthyl group, and wherein when phenyl ring A is replaced by a naphthyl group, the ethereal oxygen of Formula II and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and mare selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl (e.g., furan, thiophene, pyrrole, thiazole, isothiazole, oxazole, isoxazole, imidazole, 1,2,4-oxadiazole, 1,2,4-thiadiazole, 1,2,4-triazole, 1,2,3,-triazole, tetrazole, pyridine, pyrimidine, pyrazine, quinoline, isoquinoline, quinazoline, quinoxaline, benzothiophene, benzofuran, benzimidazole, benzisoxazole, benzisothiazole and indole) or heterocycle (e.g., imidazolidine, oxazolidine, thiazolidine, pyrrolidine, piperidine, morpholine) groups as defined below and may be further substituted by hydrogen, halo (i.e., fluorine, chlorine, bromine, iodine), (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo (i.e., chloro, fluoro, bromo or iodo), (C₁-C₄)alkyl optionally substituted with from one to three fluorine atoms, (C₁-C₄)alkoxy;
and the pharmaceutically acceptable salts thereof. Compounds of formula II, and their pharmaceutically acceptable salts, have activity in inhibiting reuptake of serotonin, dopamine, and norepinephrine.

In one embodiment, ring B is phenyl, not replaced with a naphthyl group. In another embodiment, phenyl ring B in the compounds of formula II is replaced with a naphthyl group.

In a preferred embodiment when ring B is phenyl, each Y is hydrogen or halo. In a more preferred embodiment, m is 1 or 2, and each Y is chlorine.

In another embodiment, compounds of formula II, or pharmaceutically acceptable salts, thereof are described above, but wherein X is selected from furan, thiophene, pyrrole, and 1,2,3-triazole, and wherein X may be further substituted.

In another embodiment, compounds of formula II or salts thereof are described above, but wherein each Z is selected from hydrogen and halo. Preferably, Z is hydrogen.

In a further embodiment, compounds of formula II or salts thereof are described above, wherein R³ and R⁴ are independently selected from hydrogen and unsubstituted (C₁-C₄) alkyl. Preferably, one or both of R³ and R⁴ are hydrogen.

In a further embodiment, formula II or salts thereof, wherein R¹ and R² are independently selected from hydrogen and unsubstituted (C₁-C₄)alkyl. Preferably, one of R¹ and R² is hydrogen and the other of R¹ and R² is (C₁-C₄)alkyl. More preferably, one of R¹ and R² is hydrogen and the other of R¹ and R² is methyl.

The methods and pharmaceutical compositions of this invention also relates to the pharmaceutically acceptable acid addition salts of the compounds of formula II. Examples of pharmaceutically acceptable acid addition salts of the compounds of formula II are the salts of hydrochloric acid, p-toluenesulfonic acid, fumaric acid, citric acid, succinic acid, salicylic acid, oxalic acid, hydrobromic acid, phosphoric acid, methanesulfonic acid, tartaric acid, maleic acid, di-p-toluoyl tartaric acid, acetic acid, sulfuric acid, hydroiodic acid and mandelic acid.

Unless otherwise indicated, the term "halo", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

When reference is made to SOₚ(C₁-C₆)alkyl, and p is two, this indicates a sulfone, in other words, S(=O)₂(C₁-C₆)alkyl.

When reference is made herein to a disorder or condition that can be treated by inhibiting the reuptake of serotonin, dopamine, or norepinephrine, this means that the disorder or condition has as a contributing factor at least one of serotonin, dopamine, or norepinephrine-mediated neurotransmission. The disorder or condition may have as a contributing factor one, two, or all three of the aforementioned types of neurotransmission. Moreover, a factor or factors other than serotonin, dopamine, or norepinephrine-mediated neurotransmission may also contribute to the disorder or condition. Disorders and conditions to which serotonin, dopamine, or norepinephrine-mediated neurotransmission contribute can be ascertained by those of ordinary skill in the art and include, but are not limited to, for example, addiction and substance abuse, depression, and phobia.

The compounds of formula II may have optical centers and therefore may occur in different enantiomeric configurations. The invention includes all enantiomers, diastereomers, and other stereoisomers of such compounds of formula II, as well as racemic and other mixtures thereof.

Formula II compounds also include isotopically-labeled compounds, which are identical to those recited in formula II, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹⁸F, ¹²³I and ¹²⁵I. Compounds of the present invention and pharmaceutically acceptable salts of said compounds that contain the aforementioned isotopes and/or other isotopes of other atoms are within the scope of this invention. Isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances.

Preferred embodiments of the compounds of formula II include the following compounds of the formula II and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methylamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-Dichlorophenoxy)-5-pyrimidin-2-ylbenzyl]-methylamine;
[2-(3,4-Dichlorophenoxy)-5-pyrimidin-4-ylbenzyl]-methylamine;
[2-(3,4-Dichlorophenoxy)-5-(2-methylpyrimidin-4-yl)-benzyl]-methylamine;
{1-[2-(3,4-Dichlorophenoxy)-5-(2-methylpyrimidin-4-yl)-phenyl]-ethyl}-methylamine;
4-[4-(3,4-Dichlorophenoxy)-3-(1-methylpyrrolidin-2-yl)-phenyl]-2-methylpyrimidine;
[2-(4-Chlorophenoxy)-5-(1-methyl-1H-pyrrol-3-yl)-benzyl]-dimethylamine;
[5-(1-methyl-1H-pyrrol-3-yl)-2-(naphthalen-2-yloxy)-benzyl]-dimethyl amine;
[5-Imidazol-1-yl-2-(naphthalen-2-yloxy)-benzyl]-dimethylamine;
1,5,5-Trimethyl-3-[3-methylaminomethyl-4-(naphthalen-2-yloxy)-phenyl]-imidazolidine-2,4-dione;
1-Methyl-3-[3-methylaminomethyl-4-(naphthalen-2-yloxy)-phenyl]-imidazolidine-2,4-dione;
3-[3-Methylaminomethyl-4-(naphthalen-2-yloxy)-phenyl]-thiazolidine-2,4-dione;
3-[3-Methylaminomethyl-4-(naphthalen-2-yloxy)-phenyl]-oxazolidine-2,4-dione;
3-[3-Methylaminomethyl-4-(naphthalen-2-yloxy)-phenyl]-oxazolidin-2-one;
3-[3-Methylaminomethyl-4-(naphthalen-2-yloxy)-phenyl]-thiazolidin-2-one;
1-Methyl-3-[3-methylaminomethyl-4-(naphthalen-2-yloxy)-phenyl]-imidazolidin-2-one;
1-Methyl-3-[3-methylaminomethyl-4-(naphthalen-2-yloxy)-phenyl]-tetrahydro-pyrimidin-2-one;
1-[4-(3,4-Dichlorophenoxy)-3-methylaminomethyl-phenyl]-3-methyl-tetrahydropyrimidin-2-one;
1-[4-(3,4-Dichlorophenoxy)-3-methylaminomethyl-phenyl]-3-methylimidazolidin-2-one;
3-[4-(3,4-Dichlorophenoxy)-3-methylaminomethyl-phenyl]-thiazolidin-2-one;
3-[4-(3,4-Dichlorophenoxy)-3-methylaminomethyl-phenyl]-oxazolidin-2-one;
[2-(3,4-Dichlorophenoxy)-5-(2-methylthiazol-4-yl)-benzyl]-methylamine;
[2-(3,4-Dichlorophenoxy)-5-(2-methyloxazol-4-yl)-benzyl]-methylamine;
[2-(3,4-Dichlorophenoxy)-5-(2,5-dimethyloxazol-4-yl)-benzyl]-methylamine;
[2-(3,4-Dichlorophenoxy)-5-(2,5-dimethylthiazol-4-yl)-benzyl]-methylamine;
[2-(3,4-Dichlorophenoxy)-5-(5-methyl-[1,2,4]thiadiazol-3-yl)-benzyl]-methylamine;
[2-(3,4-Dichlorophenoxy)-5-(5-methyl-[1,2,4]oxadiazol-3-yl)-benzyl]-methylamine;
[2-(3,4-Dichlorophenoxy)-5-[1,2,3]oxadiazol-4-yl-benzyl]-methylamine;
[2-(3,4-Dichlorophenoxy)-5-(5-methyl-[1,2,3]thiadiazol-4-yl)-benzyl]-methylamine;
[2-(3,4-Dichlorophenoxy)-5-(2,4-dimethyloxazol-5-yl)-benzyl]-methylamine;
[2-(3,4-Dichlorophenoxy)-5-(2,4-dimethylthiazol-5-yl)-benzyl]-methylamine;
[2-(3,4-Dichlorophenoxy)-5-[1,2,4]triazol-1-ylbenzyl]-methylamine;
[2-(3,4-Dichlorophenoxy)-5-(3-methyl-[1,2,4]triazol-1-yl)-benzyl]-methylamine;
[2-(4-Chlorophenoxy)-5-(3,5-dimethyl-[1,2,4]triazol-1-yl)-benzyl]-methylamine;
[2-(4-Chlorophenoxy)-5-tetrazol-1-ylbenzyl]-methylamine;
[2-(4-Chlorophenoxy)-5-(5-methyltetrazol-1-yl)-benzyl]-dimethylamine;
[2-(4-Chlorophenoxy)-5-[1,2,4]triazol-4-ylbenzyl]-dimethylamine;
[2-(4-Chlorophenoxy)-5-(1-methyl-1H-tetrazoi-5-yi)-benzyi]-dimethyiamine; and
{1-[2-(3,4-Dichlorophenoxy)-5-(1-methyl-1H-tetrazol-5-yl)-phenyl]-ethyl}-dimethylamine.

Suitable classes of a 5HT1a serotonergic antagonists and alpha-2-adrenergic antagonists that may be used in the compositions and methods of this invention include, among others, the following compounds:
(S)-1-(1H-indol-4-yloxy)-3-[(1-methylethyl)amino]-2-propanol ((S)-(-)-pindolol),
1-(2-methoxyphenyl)-4-(4-phthalimidobutyl)piperazine (NAN-190),
N-[2-[4-(2-methoxyphenyl)-1-piperazinyl]ethyl]-N-(2-pyridinyl)-cyclohexanecarboxamide (WAY-100635),
3-(cyclopentylpropylamino)-8-fluoro-3,4-dihydro-2H-1-benzopyran-5-carboxamide,
(3R)-3-(dicyclobutylamino)-8-fluoro-3,4-dihydro-2H-1-benzopyran-5-carboxamide (robalzotan),
1,2,3,4,10,14b-hexahydro-2-methyl-pyrazino[2,1-a]pyrido[2,3-c][2]benzazepine (mirtazapine),
2-(2,3-dihydro-1,4-benzodioxin-2-yl)-4,5-dihydro-1H-imidazole hydrochloride (idazoxan),
[8aR-(8aα,12aα,13aα)]-5,8,8a,9,10,11,12,12a,13,13a-decahydro-3-methoxy-12-(methylsulfonyl)-6H-isoquino[2,1-g][1,6]naphthyridine (delaquamine),
2-[(4,5-dihydro-1H-imidazol-2-yl)methyl]-2,3-dihydro-1-methyl-1H-iso-indole (BRL-44408), and
2-(1-ethyl-2-imidazolyl)methyl-1,4-benzodioxan (imiloxan).

### Detailed Description Of The Invention

The following references refer to novel biaryl ether derivatives useful as monoamine reuptake inhibitors that exhibit activity as a Serotonin Reuptake Inhibitor and that can be used, in combination with a 5HT1a antagonist or an alpha-2-antagonist in the pharmaceutical compositions and methods of this invention, and to methods of preparing the same: PCT application No.: PCT/IB00/01373 Filed Sept 27, 2000 and PCT application No. PCT/IB00/00108 filed Feb 2, 2000. United States Patent No. 4,018,830, issued April 19, 1997, refers to phenylthioaralkylamines and 2-phenylthiobenzylamines which are active as antiarrhythmics.

WO 97/17325, International Publication Date May 15, 1997, refers to derivatives of N,N-dimethyl-2-(arylthio)benzylamine which selectively influence serotonin transport in the central nervous system and are useful as antidepressants.

United States Patent 5,190,965, issued March 2, 1993, and United States Patent 5,430,063, issued July 4, 1995, refer to phenoxyphenyl derivatives which have utility in the treatment of depression.

United States Patent 4,161,529, issued July 17, 1979, refers to pyrrolidine derivatives that possess anticholesteremic and hypolipemic activity.

United States Provisional Application No. 60/121313, filed February 23, 1999 (see WO00/50380), refers to biaryl ethers that have activity in inhibiting reuptake of both serotonin and dopamine. The foregoing patents and patent applications are incorporated herein by reference in their entirety.

The SRI antidepressants of the formula I can be prepared as described in the following patent application, which is referred to above and incorporated herein by reference in its entirety; PCT application NO. PCT/IB00/01373 filed September 27, 2000. SRI antidepressants of Formula II can be prepared as described in the following patent application, which is referred to above and incorporated herein by reference in its entirety: PCT application No. PCT/IB00/00108 filed February 2, 2000.

The 5HT1a antagonist or an alpha-2-adrenergic antagonist that can be used, together with an SRI antidepressant agent in the pharmaceutical compositions and methods of this invention are those compounds and pharmaceutically acceptable salts described in the following references:
(S)-(-)-pindolol [(S)-1-(1H-indol-4-yloxy)-3-[(1-methylethyl)amino]-2-propanol] claimed in DE-1905881 published (09-25-69) and US-3,471,515 issued (10-07-69)
NAN-190[1-(2-methoxyphenyl)-4-(4-phthalimidobutyl)piperazine] claimed in DE-3,524,635 published (01-23-86) and US-4,585,773 issued (04-29-86).
WAY-100635 [N-[2-[4-(2-methoxyphenyl)-1-piperazinyl]ethyl]-N-(2-pyridinyl)-cyclo-hexanecarboxamide] claimed in EP-512755 published (11-11-92) and US-6,127,357 issued (10-03-2000)
3-(cyclopentylpropylamino)-8-fluoro-3,4-dihydro-2H-1-benzopyran-5-carboxamide, claimed in WO-9633710 published (10-31-96) and US-5,962,514 issued (10-05-99).
robalzotan [(3R)-3-(dicyclobutylamino)-8-fluoro-3,4-dihydro-2H-1-benzopyran-5-carbox-amide claimed in WO-95-11891 published (05-04-95) and US-5,420,151 issued (05-30-95).
mirtazapine 1,2,3,4,10,14b-hexahydro-2-methyl-pyrazino[2,1-a]pyrido[2,3-c][2]benzazepine]claimed in DE-2,614,406 published (10-14-76)
idazoxan [2-(2,3-dihydro-1,4-benzodioxin-2-yl)-4,5-dihydro-1 H-imidazole hydrochloride claimed in EP-33655 published (8-12-81) and US-4,818,764 issued (4-4-89).
delaquamine [[8aR-(8aα,12aα,13aα)]-5,8,8a,9,10,11,12,12a,13,13a-decahydro-3-methoxy-12-(methylsulfonyl)-6H-isoquino[2,1-g][1,6]naphthyridine claimed in EP-288,196 published (10-26-88) and US-4,960,891 issued (10-2-90)
BRL-44408 [2-[(4,5-dihydro-1H-imidazol-2-yl)methyl]-2,3-dihydro-1-methyl-1H-iso-indole] claimed in EP-275639 published (7-27-88) and US-4,918,083 issued (4-17-90)
imiloxan [2-(1-ethyl-2-imidazolyl)methyl-1,4-benzodioxan] claimed in US-4,302,469 issued (11-24-81).

All the foregoing patents and patent applications are incorporated herein by reference in their entirety.

This invention relates both to methods of treating sleep disorders including apnea in which the 5HT1a antagonist or an alpha-2-adrenergic antagonist and the SRI antidepressant agent, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, and the severity of the condition. Generally, in carrying out the methods of this invention, the 5HT1a antagonist or an alpha-2-adrenergic antagonist will be administered to an adult human in an amount ranging from about 0.5 to about 100 mg per day, in single or divided doses, preferably from about 1 to about 50.0 mg/day. The compounds may be administered on a regimen of up to 6 times per day, preferably 1 to 4 times per day, especially 2 times per day and most especially once daily. A suitable dosage level for the SRI antidepressant agent is about 0.05 to 1500 mg per day, preferably about 1.0 to 1000 mg per day, and especially about 2.5 to 500 mg per day. The compounds may be administered on a regimen of up to 6 times per day, preferably 1 to 4 times per day, especially 2 times per day and most especially once daily. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The 5HT1a serotonergic antagonists and the alpha-2-adrenergic antagonists and their pharmaceutically acceptable salts, and the SRI antidepressant agents and their pharmaceutically acceptable salts that are employed in the pharmaceutical compositions and methods of this invention are hereinafter also referred to as "therapeutic agents". The therapeutic agents can be administered via either the oral or parenteral route. Compositions containing both a 5HT1a antagonist or an alpha-2-adrenergic antagonist and an SRI antidepressant agent, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i.e*., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic agents of this invention, when administered separately (*i.e*., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tableting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well known to those skilled in the art.

As stated above, the 5HT1a antagonist or an alpha-2-adrenergic antagonist and the anxiolytic or SRI antidepressant agent may be formulated in a single pharmaceutical composition or alternatively in individual pharmaceutical compositions for simultaneous, separate or sequential use in accordance with the present invention.

Preferably the compositions according to the present invention, which contain both a 5HT1a antagonist or an alpha-2-adrenergic antagonist and an SRI antidepressant, as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.,* conventional tableting ingredients such as corn starch, lactose, sucrose, sorbitol, talc, stearic acid, magnesium stearate, dicalcium phosphate or gums, and other pharmaceutical diluents, *e.g.,* water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from 0.05 to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatin.

Preferred compositions for administration of a 5HT1a antagonist or an alpha-2-adrenergic antagonist or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g*., Tween™ 20, 40, 60, 80 or 85) and other sorbitans (*e.g*., Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn™, Infonutrol™, Lipofundin ™ and Lipiphysan™. The therapeutic agent may be either dissolved in a pre-mixed emulsion composition or alternatively it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising a 5HT1a antagonist or an alpha-2-adrenergic antagonist and an SRI antidepressant agent, or pharmaceutically acceptable salts of the same, which process comprises bringing a 5HT1a antagonist or an alpha-2-adrenergic antagonist and the SRI antidepressant agent (or the pharmaceutically acceptable salts of one or both of these therapeutic agents) into association with a pharmaceutically acceptable carrier or excipient.

It will be appreciated that the amount of the 5HT1a antagonist or an alpha-2-adrenergic antagonist and the SRI antidepressant agent required for use in the treatment of sleeping disorders, including sleep apneas, will vary not only with the particular compounds or compositions selected but also with the route of administration, the nature of the condition being treated, and the age and condition of the patient, and will ultimately be at the discretion of the patient's physician or pharmacist.

The *in vitro* activity of the SRI compounds used in this invention at the individual monoamine reuptake sites can be determined using rat synaptosomes or HEK-293 cells transfected with the human serotonin, dopamine or norepinephrine transporter, according to the following procedure adapted from those described by S. Snyder et al., (Molecular Pharmacology, **1971,** 7, 66-80), D.T. Wong et al., (Biochemical Pharmacology, **1973,** 22, 311-322), H. F. Bradford (Journal of Neurochemistry, **1969,** 16, 675-684) and D. J. K. Balfour (European Journal of Pharmacology, **1973,** 23, 19-26).

Synaptosomes: Male Sprague Dawley rats are decapitated and the brains rapidly removed. The cortex, hippocampi and corpus striata are dissected out and placed in ice cold sucrose buffer, 1 gram in 20 ml of buffer (the buffer is prepared using 320 mM sucrose containing 1mg/ml glucose, 0.1mM ethylenediamine tetraacetic acid (EDTA) adjusted to pH 7.4 with tris(hydroxymethyl)-aminomethane (TRIS) base). The tissues are homogenized in a glass homogenizing tube with a Teflon™ pestle at 350 rpm using a Potters homogenizer. The homogenate is centrifuged at 1000 x g for 10 min. at 4°C. The resulting supernatant is recentrifuged at 17,000 x g for 20 min. at 4°C. The final pellet is resuspended in an appropriate volume of sucrose buffer that yielded less than 10% uptake.

Cell Preparation: HEK-293 cells transfected with the human serotonin (5-HT), norepinephrine (NE) or dopamine (DA) transporter are grown in DMEM (Dulbecco's Modified Eagle Medium, Life Technologies Inc., 9800 Medical Center Dr., Gaithersburg, MD, catalog no. 11995-065)) supplemented with 10% dialyzed FBS (Fetal Bovine Serum, from Life Technologies, catalog no. 26300-053), 2 mM L-glutamine and 250 ug/ml G418 for the 5-HT and NE transporter or 2ug/ml puromycin for the DA transporter, for selection pressure. The cells are grown in Gibco triple flasks, harvested with Phosphate Buffered Saline (Life Technologies, catalog no. 14190-136) and diluted to an appropriate amount to yield less than 10% uptake.

Neurotransmitter Uptake Assay: The uptake assays are conducted in glass tubes containing 50 uL of solvent, inhibitor or 10uM sertraline, desipramine or nomifensine for the 5-HT, NE or DA assay nonspecific uptake, respectively. Each tube contains 400 uL of [³H]5-HT (5 nM final), [³H]NE (10 nM final) or [³H]DA (5 nM final) made up in modified Krebs solution containing 100 uM pargyline and glucose (1mg/ml). The tubes are placed on ice and 50 uL of synaptosomes or cells is added to each tube. The tubes are then incubated at 37° C for 7 min. (5-HT, DA) or 10 min. (NE). The incubation is terminated by filtration (GF/B filters), using a 96-well Brandel Cell Harvester, the filters are washed with modified Krebs buffer and counted using either a Wallac Model 1214 or Wallac Beta Plate Model 1205 scintillation counter.

Determination of the *in vivo* serotonin reuptake inhibition activity and potency of action for the compounds of the present invention can be made by measuring the ability of the compound to block the depletion of serotonin in the anterior cortex induced by (+/-)-para-chloroamphetamine (PCA) in the rat, according to a procedure adapted from R. W. Fuller, H. D. Snoddy and M. L. Cohen in Neuropharmacology 23: 539-544 (1984).

Generally, male, white Sprague-Dawley rats weighing 160-230 g each are assigned to either the control (vehicle) or test groups. When the test compound is administered subcutaneously (sc) at a given dose, it is co-administered with 5 mg/kg of para-chloroamphetamine (PCA). Three hours post-dose, the animals are sacrificed by decapitation and the anterior cortices are removed, wrapped in parafilm and frozen in dry ice (-78 C). When dosed orally (po), the rats are fasted the night before the experiment and then treated with the test compound at a given dose 30 minutes prior to the administration of the PCA (5 mg/kg, sc). After three hours, the animals are sacrificed and the tissues removed as above.

To determine the serotonin (5-HT) levels, the frozen tissues are homogenized with Branson sonifier in 0.5 mL of mobile phase in Eppendorf centrifuge tubes. Samples are then spun down at 11000 rpm for twenty minutes in a Sorval SH-MT rotor in a Sorval RC5C centrifuge. The supernatant thus obtained is pipetted into HPLC vials and the 5-HT levels are measured on HPLC-EC.

Interpretation of the results is as follows: Each experiment has a set of vehicle treated animals and a set of PCA-only animals. The mean 5-HT value of the PCA animals is subtracted from the mean 5-HT value of the vehicle animals. This is the signal or window of the response. The mean 5-HT value of each test group is determined, the mean of the PCA group subtracted from that, and that amount divided by the window is the per cent protection from the PCA effect for that dose. To report an ID₅₀, a line is drawn mathematically through the per cent protection values and the 50 per cent level calculated.

All of the title compounds of Formula I and II were assayed *in vitro* for serotonin, dopamine, and norepinephrine reuptake inhibition, and all had IC₅₀ values of about less than or equal to 250 nM for serotonin reuptake inhibition, about less than or equal to 1000 nM for dopamine reuptake inhibition, and about less than or equal to 1000 nM for norepinephrine reuptake inhibition.

When administered in combination, either as a single or as separate pharmaceutical composition(s), a serotonin 5HT1a antagonist or an alpha2-adrenergic antagonist and a SRI antidepressant agent, are presented in a ratio which is consistent with the manifestation of the desired effect. In particular, the ratio by weight of the 5HT1a antagonist or an alpha-2-adrenergic antagonist and the SRI antidepressant agent will suitably be between 0.001 to 1 and 1000 to 1, and especially between 0.01 to 1 and 100 to 1.

As used herein the term "mammal" includes animals of economic importance such as bovine, ovine, and porcine animals, especially those that produce meat, as well as domestic animals (*e.g*. cats and dogs), sports animals (*e.g.* horses), zoo animals and humans, the latter being preferred.

## Claims

1. A pharmaceutical composition for the treatment of sleep disorders including sleep apnea in a mammal, comprising: (a) a compound that exhibits activity, respectively, as an SRI antidepressant, or a pharmaceutically acceptable salt thereof; (b) a 5HT1a antagonist or an alpha-2-adrenergic antagonist or pharmaceutically acceptable salt thereof; and (c) a pharmaceutically acceptable carrier; wherein the active agents "a" and "b" above are present in amounts that render the composition effective in treating, respectively, sleep disorders including sleep apnea depression with increased efficacy.

2. A pharmaceutical composition according to claim 1, wherein the SRI antidepressant agent or pharmaceutically acceptable salt thereof is selected from compounds of the formula I, and their pharmaceutically acceptable salts: 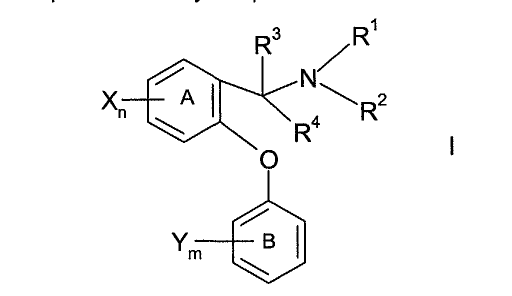 wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³ , together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X and each Y is selected, independently, from hydrogen, halo (i.e., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl wherein R⁵ is hydrogen or (C₁-C₆)alkyl, and SOₚ(C₁-C₆)alkyl wherein p is zero, one or two; and
with the proviso that: (a) no more than one of NR¹R², CR³R⁴ and R²NCR³ can form a ring; and (b) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups; or a pharmaceutically acceptable salt thereof.

3. A pharmaceutical composition according to claim 2, wherein, in the SRI antidepressant according to formula I or salt thereof, n is one, X is fluoro, R³ and R⁴ are hydrogen, R¹ is hydrogen, R² is methyl, m is two and Yₘ is 3,4-dichloro.

4. A pharmaceutical composition according to claim 2, wherein, in the SRI antidepressant according to formula I or salt thereof, m is zero, n is one, R³ and R⁴ are hydrogen, X is chloro, bromo, iodo or methyl, R¹ is hydrogen and R² is methyl.

5. A pharmaceutical composition according to claim 2, wherein said SRI antidepressant compound or salt is selected from the following compounds and their pharmaceutically acceptable salts:
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
{1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine;
{1-[2-(4-Chlorophenoxy)-5-fluorophenyl]-ethyl}-methylamine;
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fiuorophenyi]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine;
2-[2-(3,4-Dichlorophenoxy)-5-fluorophenyl]-N-methylpyrrolidine;
{1-[2-(3,4-Dichlorophenoxy)-5-fluorophenyl]-1-methylethyl}-methylamine;
{1-[2-(3,4-Dichlorophenoxy)-5-fluorophenyl]-1-methylethyl}-dimethylamine;
[4-Chloro-2-(4-chlorophenoxy)-5-fluorobenzyl]-methylamine;
[2-(3,4-Dichlorophenoxy)-5-fluoro-4-methoxybenzyl]-methylamine;
[4-(3,4-Dichlorophenoxy)-3-(dimethylaminomethyl)-phenyl]-dimethylamine
[5-Fluoro-2-(4-fluoro-3-methoxyphenoxy)-benzyl]-dimethylamine;
[2-(4-Chlorophenoxy)-5-isopropylbenzyl]-methylamine;
{1-[2-(4-Chlorophenoxy)-5-trifluoromethylphenyl]-ethyl}-methylamine;
[2-(4-Chlorophenoxy)-4,5-dimethylbenzyl]-methylamine;
{1-[5-Chloro-2(3,4-dichlorophenoxy)phenyl]-propyl}-methylamine;
[2-(3,4-Dichlorophenoxy)-5-methylsulfanyl-benzyl]-methylamine;
{1-[2-(3,4-Dichlorophenoxy)-5-methylsulfanyl-phenyl]-ethyl}-methylamine;
{1-[2-(3,4-Dichlorophenoxy)-5-methylsulfanyl-phenyl]-1-methylethyl}-methylamine; [2-(3,4-Dichlorophenoxy)-5-methylsulfanyl-benzyl]-dimethylamine;
[2-(3,4-Dichlorophenoxy)-5-methanesulfinyl-benzyl]-dimethylamine;
[2-(3,4-Dichlorophenoxy)-5-methanesulfinyl-benzyl]-methylamine;
[2-(3,4-Dichlorophenoxy)-5-methanesulfonyl-benzyl]-methylamine;
[2-(3,4-Dichlorophenoxy)-5-methanesulfonyl-benzyl]-dimethylamine;
[2-(3,4-Dichlorophenoxy)-5-(propane-2-sulfonyl)-benzyl]-methylamine;
2-[2-(3,4-Dichlorophenoxy)-5-fluorophenyl]-piperidine;
2-[2-(3,4-Dichlorophenoxy)-5-fluorophenyl]-1-methyl-piperidine;
3-[2-(3,4-Dichlor-phenoxy)-5-fluorophenyl]-4-methyl-morpholine;
2-[2-(3,4-Dichlorophenoxy)-5-fluorophenyl]-1,2-dimethyl-piperidine;
{1-[2-(3,4-Dichlorophenoxy)-5-fluorophenyl]-cyclopropyl}-dimethylamine;
2-[2-(3,4-Dichlorophenoxy)-5-fluorophenyl]-1,5-dimethyl-pyrrolidine;
3-[2-(3,4-Dichlorophenoxy)-5-fluorophenyl]-4-methyl-thiomorpholine;
{1-[2-(3,4-Dichlorophenoxy)-5-fluorophenyl]-cyclopentyl}-methylamine;
{1-[2-(3,4-Dichlorophenoxy)-5-(propane-2-sulfonyl)-phenyl]-ethyl}-methylamine; and
[4-Chloro-2-(3,4-dichlorophenoxy)-5-methanesulfonyl-benzyl]-methylamine.

6. A pharmaceutical composition according to claim 1, wherein the SRI antidepressant agent or pharmaceutically acceptable salt thereof is selected from compounds of the formula II, as defined below, and their pharmaceutically acceptable salts: 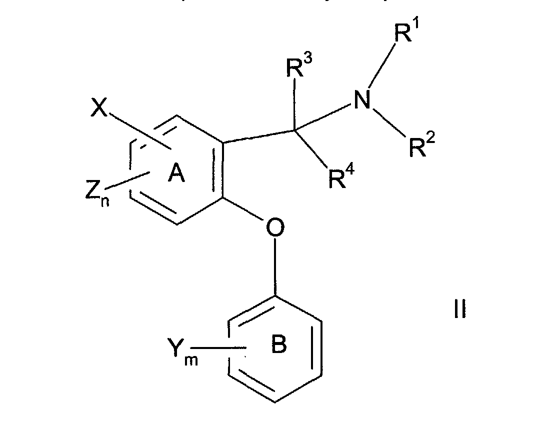 wherein phenyl ring A and phenyl ring B can each, independently, be replaced by a naphthyl group, and wherein when phenyl ring A is replaced by a naphthyl group, the ethereal oxygen of formula II and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocycle, and wherein each X may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, (C₁-C₄)alkoxy; or a pharmaceutically acceptable salt thereof.

7. A pharmaceutical composition according to claim 6, wherein, in the SRI antidepressant according to formula II or salt thereof, ring B is phenyl, not replaced with a naphthyl group.

8. A pharmaceutical composition according to claim 6, wherein, in the SRI antidepressant according to formula II or salt thereof, each Y is hydrogen or halo.

9. A pharmaceutical composition according to claim 7, wherein, in the SRI antidepressant according to formula II or salt thereof, m is 1 or 2, and wherein each Y is chlorine.

10. A pharmaceutical composition according to claim 6, wherein, in the SRI antidepressant according to formula II or salt thereof, X is selected from furan, thiophene, pyrrole, and 1,2,3-triazole, and wherein X may be further substituted.

11. A pharmaceutical composition according to claim 6, wherein, in the SRI antidepressant according to formula II or salt thereof, each Z is selected from hydrogen and halo.

12. A pharmaceutical composition according to claim 11, wherein, in the SRI antidepressant according to formula II or salt thereof, each Z is hydrogen.

13. A pharmaceutical composition according to claim 6, wherein, in the SRI antidepressant according to formula II or salt thereof, R³ and R⁴ are independently selected from hydrogen and unsubstituted (C₁-C₄) alkyl.

14. A pharmaceutical composition according to claim 13, wherein, in the SRI antidepressant according to formula II or salt thereof, one or both of R³ and R⁴ are hydrogen.

15. A pharmaceutical composition according to claim 6, wherein, in the SRI antidepressant according to formula II or salt thereof, R¹ and R² are independently selected from hydrogen and unsubstituted (C₁-C₄)alkyl.

16. A pharmaceutical composition according to claim 15, wherein, in the SRI antidepressant according to formula II or salt thereof, one of R¹ and R² is hydrogen and the other of R¹ and R² is (C₁-C₄)alkyl.

17. A pharmaceutical composition according to claim 15, wherein, in the SRI antidepressant according to formula II or salt thereof, one of R¹ and R² is hydrogen and the other of R¹ and R² is methyl.

18. A pharmaceutical composition to claim 6, wherein the SRI antidepressant is selected from the group consisting of:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methylamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethyl-phenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-Dichlorophenoxy)-5-pyrimidin-2-ylbenzyl]-methylamine;
[2-(3,4-Dichlorophenoxy)-5-pyrimidin-4-ylbenzyl]-methylamine;
[2-(3,4-Dichlorophenoxy)-5-(2-methylpyrimidin-4-yl)-benzyl]-methylamine;
{1-[2-(3,4-Dichlorophenoxy)-5-(2-methylpyrimidin-4-yl)-phenyl]-ethyl}-methylamine;
4-[4-(3,4-Dichlorophenoxy)-3-(1-methylpyrrolidin-2-yl)-phenyl]-2-methylpyrimidine;
[2-(4-Chlorophenoxy)-5-(1-methyl-1H-pyrrol-3-yl)-benzyl]-dimethylamine;
[5-(1-methyl-1H-pyrrol-3-yl)-2-(naphthalen-2-yloxy)-benzyl]-dimethyl amine;
[5-lmidazol-1-yl-2-(naphthalen-2-yloxy)-benzyl]-dimethylamine;
1,5,5-Trimethyl-3-[3-methylaminomethyl-4-(naphthalen-2-yloxy)-phenyl]-imidazolidine-2,4-dione;
1-Methyl-3-[3-methylaminomethyl-4-(naphthalen-2-yloxy)-phenyl]-imidazolidine-2,4-dione;
3-[3-Methylaminomethyl-4-(naphthalen-2-yloxy)-phenyl]-thiazolidine-2,4-dione;
3-[3-Methylaminomethyl-4-(naphthalen-2-yloxy)-phenyl]-oxazolidine-2,4-dione;
3-[3-Methylaminomethyl-4-(naphthalen-2-yloxy)-phenyl]-oxazolidin-2-one;
3-[3-Methylaminomethyl-4-(naphthalen-2-yloxy)-phenyl]-thiazolidin-2-one;
1-Methyl-3-[3-methylaminomethyl-4-(naphthalen-2-yloxy)-phenyl]-imidazolidin-2-one;
1-Methyl-3-[3-methylaminomethyl-4-(naphthalen-2-yloxy)-phenyl]-tetrahydro-pyrimidin-2-one;
1-[4-(3,4-Dichlorophenoxy)-3-methylaminomethyl-phenyl]-3-methyl-tetrahydropyrimidin-2-one;
1-[4-(3,4-Dichlorophenoxy)-3-methylaminomethyl-phenyl]-3-methylimidazolidin-2-one; 3-[4-(3,4-Dichlorophenoxy)-3-methylaminomethyl-phenyl]-thiazolidin-2-one;
3-[4-(3,4-Dichlorophenoxy)-3-methylaminomethyl-phenyl]-oxazolidin-2-one;
[2-(3,4-Dichlorophenoxy)-5-(2-methylthiazol-4-yl)-benzyl]-methylamine;
[2-(3,4-Dichlorophenoxy)-5-(2-methyloxazol-4-yl)-benzyl]-methylamine;
[2-(3,4-Dichlorophenoxy)-5-(2,5-dimethyloxazol-4-yl)-benzyl]-methylamine;
[2-(3,4-Dichlorophenoxy)-5-(2,5-dimethylthiazol-4-yl)-benzyl]-methylamine;
[2-(3,4-Dichlorophenoxy)-5-(5-methyl-[1,2,4]thiadiazol-3-yl)-benzyl]-methylamine;
[2-(3,4-Dichlorophenoxy)-5-(5-methyl-[1,2,4]oxadiazol-3-yl)-benzyl]-methylamine;
[2-(3,4-Dichlorophenoxy)-5-[1,2,3]oxadiazol-4-yl-benzyl]-methylamine;
[2-(3,4-Dichlorophenoxy)-5-(5-methyl-[1,2,3]thiadiazol-4-yl)-benzyl]-methylamine;
[2-(3,4-Dichlorophenoxy)-5-(2,4-dimethyloxazol-5-yl]-benzyl]-methylamine;
[2-(3,4-Dichlorophenoxy)-5-(2,4-dimethylthiazol-5-yl)-benzyl]-methylamine;
[2-(3,4-Dichlorophenoxy)-5-[1,2,4]triazol-1-ylbenzyl]-methylamine;
[2-(3,4-Dichlorophenoxy)-5-(3-methyl-[1,2,4)triazol-1-yl)-benzyl]-methylamine;
[2-(4-Chlorophenoxy)-5-(3,5-dimethyl-[1,2,4]triazol-1-yl)-benzyl]-methylamine;
[2-(4-Chlorophenoxy)-5-tetrazol-1-ylbenzyl]-methylamine;
[2-(4-Chlorophenoxy)-5-(5-methyltetrazol-1-yl)-benzyl]-dimethylamine;
[2-(4-Chlorophenoxy)-5-[1,2,4]triazol-4-ylbenzyl]-dimethylamine;
[2-(4-Chlorophenoxy)-5-(1-methyl-1H-tetrazol-5-yl)-benzyl]-dimethylamine; and
{1-[2-(3,4-Dichlorophenoxy)-5-(1 -methyl-1H-tetrazol-5-yl)-phenyl]-ethyl}-dimethylamine,
and pharmaceutically acceptable salts thereof.

19. A pharmaceutical composition according to claim 1 wherein the 5HT1a antagonist or alpha-2-adrenergic antagonist or a pharmaceutically acceptable salt thereof is selected from:
(S)-1-(1H-indol-4-yloxy)-3-[(1-methylethyl)amino]-2-propanol ((S)-(-)-pindolol),
1-(2-methoxyphenyl)-4-(4-phthalimidobutyl)piperazine (NAN-190),
N-[2-[4-(2-methoxyphenyl)-1-piperazinyl]ethyl]-N-(2-pyridinyl)-cyclohexanecarboxamide (WAY-100635),
3-(cyclopentylpropylamino)-8-fluoro-3,4-dihydro-2H-1-benzopyran-5-carboxamide,
(3R)-3-(dicyclobutylamino)-8-fluoro-3,4-dihydro-2H-1-benzopyran-5-carboxamide (robalzotan),
1,2,3,4,10,14b-hexahydro-2-methyl-pyrazino[2,1-a]pyrido[2,3-c][2]benzazepine (mirtazapine),
2-(2,3-dihydro-1,4-benzodioxin-2-yl)-4,5-dihydro-1H-imidazole hydrochloride (idazoxan),
[8aR-(8aα,12aα,13aα)]-5,8,8a,9,10,11,12,12a,13,13a-decahydro-3-methoxy-12-(methylsulfonyl)-6H-isoquino[2,1-g][1,6]naphthyridine (delaquamine),
2-[(4,5-dihydro-1 H-imidazol-2-yl)methyl]-2,3-dihydro-1-methyl-1H-iso-indole (BRL-44408), and
2-(1-ethyl-2-imidazolyl)methyl-1,4-benzodioxan (imiloxan).

20. A pharmaceutical composition according to claim 1 wherein the amount of the SRI antidepressant, or pharmaceutically acceptable salt thereof, in said composition is from about 0.05 mg to about 1500 mg and the amount of the 5HT1a antagonist or an alpha-2-adrenergic antagonist or pharmaceutically acceptable salt thereof is from about 0.5 mg to about 100 mg.

21. A pharmaceutical composition according to claim 20 wherein the amount of the SRI antidepressant, or pharmaceutically acceptable salt thereof, in said composition is from about 2.5 mg to about 500 mg and the amount of the 5HT1 a antagonist or an alpha-2-adrenergic antagonist or pharmaceutically acceptable salt thereof is from about 1.0 mg to about 50 mg.

22. The use of: (a) a compound that exhibits activity as an SRI antidepressant, or a pharmaceutically acceptable salt thereof; and (b) a 5HT1a antagonist or an alpha-2-adrenergic antagonist or pharmaceutically acceptable salt thereof; for the manufacture of a medicament for treating sleep disorder including sleep apnea in a mammal.

23. The use according to claim 22, wherein the SRI antidepressant or pharmaceutically acceptable salt thereof is as defined in any of claims 2 to 18.

24. The use according to either of claims 22 and 23, wherein the 5HT1a antagonist or an alpha-2-adrenergic antagonist or pharmaceutically acceptable salt thereof is as defined in claim 19.

25. The use according to any of claims 22 to 24, wherein the amount of the SRI antidepressant, or pharmaceutically acceptable salt thereof, and the amount of the 5HT1a antagonist or an alpha-2-adrenergic antagonist or pharmaceutically acceptable salt thereof are as defined in either of claims 20 and 21.

26. The use according to any of claims 22 to 25, wherein the SRI antidepressant or pharmaceutically acceptable salt thereof, and the 5HT1a antagonist or an alpha-2-adrenergic antagonist or pharmaceutically acceptable salt thereof, are parts of the same dosage form.
